# EUROPÄISCHE PATENTSCHRIFT

(11) **EP 2 629 702 B1**
(45) Veröffentlichungstag und Bekanntmachung des Hinweises auf die Patenterteilung: **14.09.2016**
(21) Anmeldenummer: 11819073.5
(22) Anmeldetag: 12.10.2011
(51) Int. Cl.: A61F 2/36, A61F 2/32

(54) **VORMONTIERTES GELENKPROTHESENSYSTEMTEIL**
PREMOUNTED JOINT PROSTHESIS SYSTEM PART
PARTIE PRÉMONTÉE DE SYSTÈME DE PROTHÈSE D'ARTICULATION

(30) Priorität: 18.10.2010 DE 202010008828 U
(43) Veröffentlichungstag der Anmeldung: 28.08.2013
(73) Patentinhaber: Merete Holding GmbH, 10719 Berlin (DE)
(72) Erfinder: ANAPLIOTIS, Emmanuel, 14195 Berlin (DE)
(74) Vertreter: Bittner, Thomas L.
(86) Internationale Anmeldenummer: PCT/DE2011/075250
(87) Internationale Veröffentlichungsnummer: WO 2012/059098

(56) Entgegenhaltungen:
- EP-A1- 1 099 426
- WO-A1-2005/089676
- WO-A2-2011/045737
- AT-B- 413 260
- FR-A1- 2 879 917

## Beschreibung

Die Erfindung bezieht sich auf ein modulares Gelenkprothesensystem, insbesondere modulares Hüftgelenkprothesensystem, ein vormontiertes Gelenkprothesensystemteil und einen Sterilartikel.

### Hintergrund der Erfindung

Gelenkprothesensysteme sind in verschiedenen Ausführungsformen bekannt. Üblicherweise verfügen modulare Systeme über eine Gelenkpfanne, die mit einer Kugelaufnahme für einen Gelenkkugelkopf gebildet ist. Der Gelenkkugelkopf seinerseits wird beim Einsatz mit einem Prothesenhals verbunden, was direkt oder mit Hilfe von Zwischenelementen erfolgen kann. Auf diese Weise wird eine starre Verbindung zwischen dem Prothesenhals und dem Gelenkkugelkopf hergestellt. Der Gelenkkugelkopf selbst ist frei beweglich in der Gelenkpfanne aufgenommen.

Aus dem Dokument DE 91 03 574 U1 ist ein modulares Gelenkprothesensystem bekannt, welches aus einem Zwischenelement, einer Gelenkkugel sowie einem Prothesenschaft mit einem Hals zur Aufnahme der Gelenkkugel besteht. Das Zwischenelement ist zwischen den Prothesenschaft und die Gelenkkugel einsetzbar. Die Außenkontur des Zwischenelementes ist an die Innenkontur einer Sackbohrung in der Gelenkkugel angepasst. Die Innenkontur des Zwischenelementes entspricht der Außenkontur des Prothesenhalses. Die Innen- und die Außenkontur des Zwischenelementes sind nicht axial zueinander gebildet.

Aus dem Dokument WO 2005/089676 A1 ist weiterhin ein modulares Gelenkprothesensystem bekannt, bei dem zwischen Prothesenschaft und Gelenkkugel mehrere Zwischenelemente angeordnet sind, die ineinander gesteckt werden.

Modulare Gelenkprothesensysteme der vorgenannten Art werden beispielsweise unter dem Handelsnamen BioBall angeboten. Sie ermöglichen es, den Mittelpunkt des Gelenkkugelkopfes relativ zur Längsachse des Prothesenhalses zu versetzen. Auf diese Weise ist eine individuelle Anpassung für den Patienten ermöglicht.

In Verbindung mit Hüftendprothesenoperationen führen häufig nachoperative Luxationen zu Problemen, die zu schwerwiegenden Komplikationen führen können. Es besteht deshalb Bedarf für verbesserte Gelenkprothesensysteme.

Das Dokument FR 2 879917 A1 offenbart eine Prothese für ein Hüftgelenk. Die Hüftprothese umfasst einen Schaft, der eine Epiphysis aufnimmt, wobei die Epiphysis konfiguriert ist, einen Gelenkkopf aufzunehmen. Die Epiphysis weist an einem Ende eine konische Oberfläche auf. Des Weiteren ist eine Gelenkpfanne für die Hüftprothese offenbart, die eine zweifache Mobilität ermöglicht. Die Gelenkpfanne weist eine Außenschale und ein Inlay auf, wobei das Inlay beweglich in der Außenschale angeordnet ist.

Das Dokument WO 2005/089676 A1 offenbart ein modulares Gelenkprothesensystem, bestehend aus einem Prothesenschaft mit einem Hals, einem Außenkonus und einem ersten Zwischenelement, das zwischen dem Außenkonus des Prothesenschafts und einem Innenkonus der Gelenkkugel einfügbar ist.

Das Dokument EP 1 099 426 A1 offenbart eine weitere Prothese mit einer zweifachen Mobilität.

In dem Dokument WO 2011/045737 A2 ist eine Hüftgelenkspfanne offenbart. Ein Kugelkopf ist in einem Inlay angeordnet, das wiederum von einer Gelenkpfanne aufgenommen ist.

### Zusammenfassung der Erfindung

Aufgabe der Erfindung ist es ein verbesserte Technologien für ein modulares Gelenkprothesensystem, insbesondere ein modulares Hüftgelenkprothesensystem, anzugeben, welche die individuelle Anpassbarkeit des Prothesensystems an den Patienten ermöglichen und darüber hinaus die Wahrscheinlichkeit für Luxationen mindern. Darüber hinaus soll die Wahrscheinlichkeit für das Auftreten von Komplikationen beim und / oder nach dem Implantieren der Gelenkprothese im Patientenkörper gemindert werden.

Diese Aufgabe wird erfindungsgemäß durch vormontiertes Gelenkprothesensystemteil und ein Sterilartikel nach den unabhängigen Ansprüchen 1 und 8 gelöst. Vorteilhafte Ausgestaltungen der Erfindung sind Gegenstand von abhängigen Unteransprüchen.

Es ist ein modulares Gelenkprothesensystem offenbart, insbesondere modulares Hüftgelenkprothesensystem, mit:
- einem Zwischenelement, welches einen Innenkonus zum Aufnehmen eines Prothesenhalses und einen Außenkonus aufweist,
- einem Gelenkkugelkopf, welcher als Steckkopf mit einem dem Außenkonus des Zwischenelementes zugeordneten Innenkonus gebildet ist, und
- einer bipolaren Gelenkpfanne, bei der ein Inlay frei beweglich in einer Außenschale aufgenommen ist und die eine dem Gelenkkugelkopf zugeordnete Kugelaufnahme aufweist, die konfiguriert ist, den Gelenkkugelkopf beweglich aufzunehmen.

Nach einem weiteren Aspekt der Erfindung ist ein vormontiertes Gelenkprothesensystemteil für ein modulares Gelenkprothesesystem, insbesondere ein Gelenkprothesensystem der vorgenannten Art, mit den folgenden Merkmalen geschaffen:
- einem einer bipolaren Gelenkpfanne zugeordneten Inlay aus einem Kunststoffmaterial, welches mit einer Kugelaufnahme gebildet und konfiguriert ist, frei beweglich in einer Außenschale der bipolaren Gelenkpfanne aufgenommen zu werden, und
- einem Gelenkkugelkopf, welcher als Steckkopf beweglich in der Kugelaufnahme des Inlays angeordnet und mit einem Innenkonus gebildet ist.

Schließlich umfasst die Erfindung den Gedanken eines Sterilartikels mit einem vormontierten Gelenkprothesensystemteil, welches in einer sterilen Verpackung aufgenommen ist.

Das vorgeschlagene Gelenkprothesensystem vereinigt in sich sowohl die individuelle Anpassbarkeit des Prothesensystems an unterschiedliche Patienten, was insbesondere mit Hilfe des Zwischenelementes und dessen konkreter Ausprägung ermöglicht ist, als auch den Vorteil einer verminderten Luxationswahrscheinlichkeit, dadurch, dass die Gelenkpfanne, welche dem Gelenkkugelkopf aufnimmt, bipolar ausgebildet ist. Die bipolare Ausführung bedeutet insbesondere, dass in einer Außenschale ein Inlay frei beweglich aufgenommen ist, welches seinerseits den Gelenkkugelkopf beweglich aufnimmt. Hierbei schnappt der Gelenkkugelkopf in der zugeordneten Kugelaufnahme ein. Die Montage des Gelenkkugelkopfes erfolgt bevorzugt mittels eines Handpresswerkzeuges, mit dem der Gelenkkugelkopf in die Kugelaufnahme eingepresst wird. Es sind hierdurch insgesamt einerseits eine feste Verbindung zwischen Gelenkkugelkopf und Aufnahme and andererseits eine Art doppelte Beweglichkeit zur Verfügung gestellt. Die Beweglichkeit des Inlays innerhalb der Außenschale führt zu einer deutlichen Reduktion der Scherkräfte an der Kontaktfläche zwischen Knochen und Außenschale und wirkt sich so günstig auf eine langfristige Stabilität des Implantats aus. Die im Zusammenhang mit der Nutzung des Zwischenelementes als solche bekannten Vorteile, insbesondere die individuelle Anpassbarkeit an den Patienten und die sich hieraus ergebende Reduzierung nachoperativer Probleme, kommen aufgrund der Nutzung der bipolaren Gelenkpfanne in optimierter Art und Weise zur Entfaltung.

Mit Hilfe des vormontierten Gelenkprothesensystemteiles ist ein Modul für die Gelenkprothese zur Verfügung gestellt, dessen Nutzung es dem Arzt beim Implantieren ermöglicht, insbesondere den Schritt zum Einpressen des Gelenkkugelkopfes in die Kugelaufnahme des Inlays im Operationsfeld, was regelmäßig mit einer Handpresse ausgeführt wird, einzusparen. Das vormontierte Gelenkprothesensystemteil kann in einer sterilen Verpackung bereitgestellt werden. Kontaminationen, die auftreten können, wenn bei bekannten Systemen beim Implantieren vor Ort der Gelenkkugelkopf eingepresst wird, sind ausgeschlossen.

Eine vorteilhafte Ausführungsform der Erfindung sieht vor, dass der Innenkonus und der Außenkonus des Zwischenelements zueinander nicht koaxial gebildet sind.

Eine bevorzugte Weiterbildung der Erfindung sieht vor, dass das Zwischenelement mehrstückig ausgeführt ist, mehrere Teilzwischenelemente jeweils einen Innenkonus und einen Außenkonus aufweisen. Es kann hierbei in einer Ausführung vorgesehen sein, dass die mehreren Teilzwischenelemente jeweils einen Innenkonus und einen Außenkonus aufweisen, die zueinander nicht koaxial angeordnet sind. Auch können Zwischenelemente kombiniert werden, bei denen für ein Zwischenelement Innen- und Außenkonus koaxial und für ein weiteres Zwischenelement Innen- und Außenkonus nicht koaxial gebildet sind. Beispielsweise sind zwei Teilzwischenelemente vorgesehen, die jeweils über einen Innenkonus und einen Außenkonus verfügen. Bei beiden Teilzwischenelementen sind Innen- und Außenkonus zueinander nicht koaxial. Der Innenkonus des einen Teilzwischenelementes ist hinsichtlich seiner Form an eine Aufnahme des Außenkonus des anderen Teilzwischenelementes angepasst. Die Mehrstückigkeit des Zwischenelementes vergrößert die Flexibilität bei der individuellen Anpassung des Gelenkprothesensystems an die Verhältnisse des Patienten. Alternativ kann vorgesehen sein, dass das Zwischenelement einstückig ausgeführt ist. Zur individuellen Anpassbarkeit an den Patienten können in einem Bausatz Zwischenelemente mit unterschiedlicher Länge bereitgestellt werden. Auch kann vorgesehen sein, dass die Längsrichtungen in einem unteren und einem oberen Abschnitt des Zwischenelementes einen Winkel zueinander einnehmen, so dass beispielsweise der obere Abschnitt im Vergleich zum unteren Abschnitt abgeknickt ist.

Bei einer zweckmäßigen Ausgestaltung der Erfindung kann vorgesehen sein, dass die Außenschale aus Metall ist. Die Außenschale der Gelenkpfanne kann für eine zementierte oder nichtzementierte Implantierung konfiguriert sein.

Eine vorteilhafte Ausführungsform der Erfindung sieht vor, dass das Inlay aus einem Kunststoffmaterial ist. Als Kunststoffmaterial kann beispielsweise Polyethylen zum Einsatz kommen.

Bevorzugt sieht eine Fortbildung der Erfindung vor, dass der Innenkonus am Zwischenelement in einer Sackbohrung gebildet ist. Die Sackbohrung dient zur mechanisch festen Aufnahme des Prothesenhalses.

Bei einer vorteilhaften Ausgestaltung der Erfindung kann vorgesehen sein, dass an der Außenschale ein oder mehrere Befestigungselemente gebildet sind. Als Befestigungselemente können beispielsweise Befestigungszapfen, die von der äußeren Oberfläche der Außenschale abstehen, und / oder Schraubenlaschen vorgesehen sein. Letztere dienen dazu, die Gelenkpfanne mit Hilfe einer oder mehrerer Schrauben am Knochen zu befestigen.

Eine Weiterbildung der Erfindung kann vorsehen, dass der Gelenkkugelkopf aus einem Keramikmaterial ist. Alternativ kann der Gelenkkugelkopf aus einem Metallmaterial bestehen.

Im Zusammenhang mit vorteilhaften Ausgestaltungen des vormontierten Gelenkprothesensystemteils gelten die in Verbindung mit zweckmäßigen Ausführungen des Gelenkprothesensystems gemachten Erläuterungen entsprechend. Insbesondere kann vorgesehen sein, dass das Inlay aus Polyethylen besteht. In einer Ausgestaltung weist das Zwischenelement ein Kopplungselement auf, welches konfiguriert ist, lösbar an ein Wechselwerkzeug zu koppeln.

Auch kann das vormontierte Gelenkprothesensystemteil mit einem Zwischenelement gebildet sein, bei dem ein Außenkonus in dem zugeordneten Innenkonus des Gelenkkugelkopfes lösbar angeordnet ist und das einen Innenkonus zum Aufnehmen eines weiteren Zwischenelementes oder eines Prothesenhalses aufweist.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1: eine schematische Darstellung von Elementen eines Hüftgelenkprothesensystems, nämlich eine Hüftprothese, bei der auf einen Prothesenhals ein Zwischenelement aufgesetzt ist, welches seinerseits in einem Gelenkkugelkopf aufgenommen ist,
- Fig. 2: eine schematische Darstellung eines Hüftgelenkprothesensystems, bei dem der Gelenkkugelkopf in einer bipolaren Gelenkpfanne aufgenommen ist,
- Fig. 3: eine schematische Darstellung eines vormontierten Gelenkprothesensystemteils für ein bipolares Gelenkprothesensystem wie es beispielhaft in Fig. 2 gezeigt ist, und
- Fig. 4: eine schematische Darstellung eines weiteren vormontierten Gelenkprothesensystemteils für ein bipolares Gelenkprothesensystem wie es beispielhaft in Fig. 2 gezeigt ist.

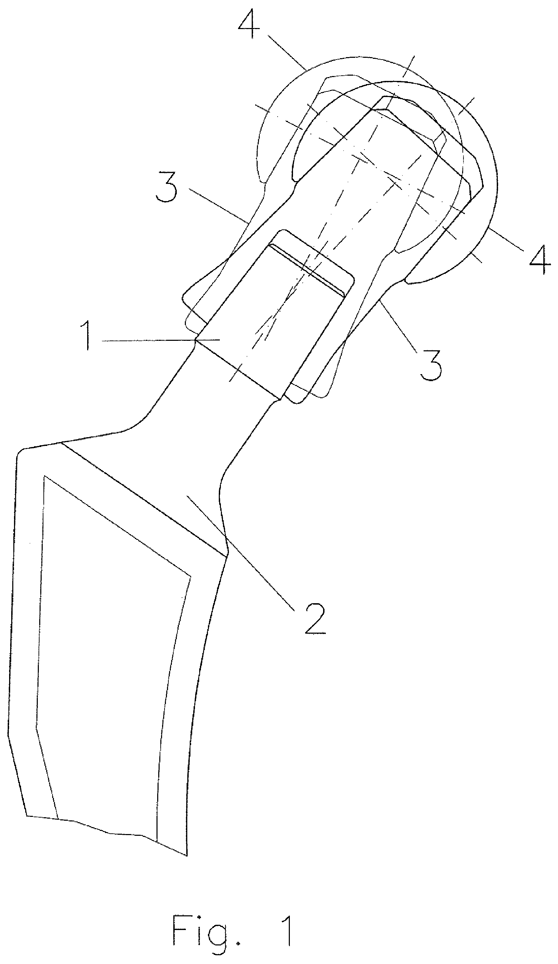

Fig. 1 zeigt eine schematische Darstellung von Elementen eines Hüftgelenkprothesensystems, bei der auf einen Prothesenhals 1 einer Hüftgelenkprothese 2 ein Zwischenelement 3 aufgesteckt ist, welches seinerseits in einen Gelenkkugelkopf 4 eingesteckt ist. Die Darstellung zeigt die Verwendung von zwei unterschiedlichen Zwischenelementen 3, die auf unterschiedliche Art und Weise dafür sorgen, dass der Mittelpunkt des Gelenkkugelkopfes 4 seitlich versetzt zur Längsrichtung des Prothesenhalses 2 angeordnet ist. Unterschiedliche Ausführungsarten des Zwischenelementes 3, die in Ausgestaltungen der Erfindung zur Anwendung kommen können, sind als solche zum Beispiel in den Dokumenten DE 91 03 574 U1 und WO 2005 / 089676 A1 offenbart. Sämtliche dort beschriebenen Ausgestaltungen können in vorteilhaften Weiterbildungen der vorliegenden Erfindung eingesetzt werden. Ein- oder mehrteilige Zwischenelemente können zum Einsatz kommen. Die Zwischenelemente verfügen jeweils über einen Innen- und einen Außenkonus, die koaxial oder nicht koaxialen gebildet sein können. Fig. 1 zeigt Ausführungsformen, wo Innen- und Außenkonus des Zwischenelementes nicht koaxial gebildet sind.

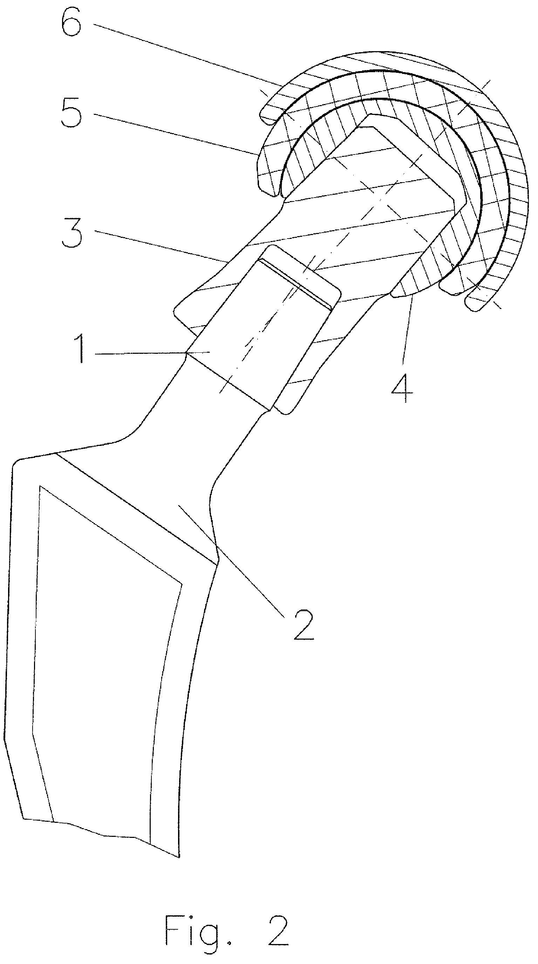

Fig. 2 zeigt nun eine schematische Darstellung eines Hüftgelenkprothesensystems, bei dem der Gelenkkugelkopf 4 beweglich in einem Inlay 5 aufgenommen ist, wobei das Inlay 5 seinerseits frei beweglich in einer Außenschale 6 angeordnet ist. Das Inlay 5 und die Außenschale 6 bilden eine bipolare Gelenkpfanne. Auf diese Weise ist eine vergrößerte Beweglichkeit des Hüftgelenkprothesensystems ermöglicht, da sowohl der Gelenkkugelkopf 4 innerhalb des Inlays 5 als auch das Inlay 5 selbst in der Außenschale 6 beweglich sind. Das Hüftgelenkprothesensystem ermöglicht aufgrund der bipolaren Gelenkpfanne eine verbesserte Ausnutzung der Anpassungsoptionen, die sich aus der Verwendung des Zwischenelementes 3 ergeben.

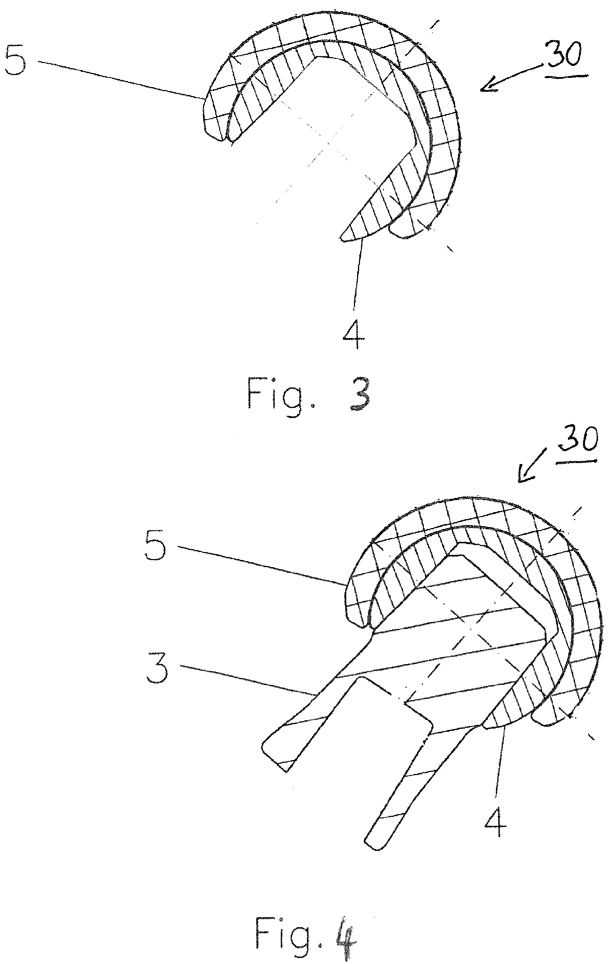

Fig. 3 und 4 zeigen eine schematische Darstellung eines vormontierten Gelenkprothesensystemteils 30 für ein bipolares Gelenkprothesensystem wie es beispielhaft in Fig. 2 gezeigt ist. Das so vormontierte Gelenkprothesensystemteil 30 kann außerhalb des Operationsfeldes in einer Reinraumumgebung montiert und dann steril verpackt werden.

Bei der Ausführung des vormontierten Gelenkprothesensystemteils 30 in Fig. 3 ist der Gelenkkugelkopf 4 als Steckkopf beweglich in der Kugelaufnahme des Inlays 5, welches aus einem Kunststoffmaterial besteht, angeordnet und so für die bei der Operation selbst noch stattfindende Aufnahme des Prothesenhalses 2, sei es direkt oder vermittelt über ein oder mehrere Zwischenelemente 3, vorbereitet.

Bei der Ausgestaltung des vormontierten Gelenkprothesensystemteils 30 in Fig. 4 ist der Gelenkkugelkopf 4 als Steckkopf beweglich in der Kugelaufnahme des Inlays 5 angeordnet und nimmt zusätzlich bereits das Zwischenelement 3 lösbar auf.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungen von Bedeutung sein.

## Patentansprüche

1. Gelenkprothesensystemteil für ein modulares Gelenkprothesensystem, mit:
- einem einer bipolaren Gelenkpfanne zugeordneten Inlay (5) aus einem Kunststoffmaterial, welches mit einer Kugelaufnahme gebildet und konfiguriert ist, frei beweglich in einer Außenschale (6) der bipolaren Gelenkpfanne aufgenommen zu werden, und
- einem Gelenkkugelkopf (4), welcher mit einem Innenkonus gebildet ist, wobei der Gelenkkugelkopf (4) als Steckkopf beweglich in der Kugelaufnahme des Inlays (5) vormontiert ist,
**gekennzeichnet durch** ein Zwischenelement (3), bei dem ein Außenkonus in dem zugeordneten Innenkonus des vormontierten Gelenkkugelkopfes (4) lösbar angeordnet ist und das einen Innenkonus zum Aufnehmen eines weiteren Zwischenelementes oder eines Prothesenhalses (1) aufweist.

2. Gelenkprothesensystemteil nach Anspruch 1, **dadurch gekennzeichnet, dass** das Inlay (5) aus Polyethylen besteht.

3. Gelenkprothesensystemteil nach Anspruch 1 oder 2, **dadurch gekennzeichnet, dass** der Innenkonus und der Außenkonus zueinander nicht koaxial gebildet sind.

4. Gelenkprothesensystemteil nach mindestens einem der vorangehenden Ansprüche, **dadurch gekennzeichnet, dass** das Zwischenelement (3) ein Kopplungselement aufweist, welches konfiguriert ist, lösbar an ein Wechselwerkzeug zu koppeln.

5. Gelenkprothesensystemteil nach mindestens einem der vorangehenden Ansprüche, **dadurch gekennzeichnet, dass** der Innenkonus am Zwischenelement (3) in einer Sackbohrung gebildet ist.

6. Gelenkprothesensystemteil nach mindestens einem der vorangehenden Ansprüche, **dadurch gekennzeichnet, dass** der Gelenkkugelkopf (4) aus einem Keramikmaterial ist.

7. Sterilartikel, mit einem Gelenkprothesensystemteil nach mindestens einem der Ansprüche 1 bis 6, welches in einer sterilen Verpackung aufgenommen ist.

## Claims

1. A joint prosthesis system part for a modular joint prosthesis system, comprising:
- an inlay (5) made of a plastic material which is associated with a bipolar joint socket, wherein the inlay (5) is formed with a ball receptacle and is configured so as to be freely movably received in an outer shell (6) of the bipolar joint socket, and
- a joint ball head (4) which is movably disposed as a plug head in the ball receptacle of the inlay (5) and which is formed with an inner cone,
**characterized by** an intermediate element (3), wherein an outer cone is releasably disposed in the associated inner cone of the joint ball head (4) and has an inner cone to receive a further intermediate element or a prosthesis neck (1).

2. The joint prosthesis system part as claimed in claim 1, **characterized in that** the inlay (5) is made of polyethylene.

3. The joint prosthesis system part as claimed in claim 1 or 2, **characterized in that** the inner cone and the outer cone are not formed coaxially with respect to one another.

4. The joint prosthesis system part as claimed in at least one of the preceding claims, **characterized in that** the intermediate element (3) has a coupling element which is configured so as to be coupled to a changing tool.

5. The joint prosthesis system part as claimed in at least one of the preceding claims, **characterized in that** the inner cone is formed on the intermediate element (3) in a blind bore.

6. The joint prosthesis system part as claimed in at least one of the preceding claims, **characterized in that** the joint ball head (4) is made of a ceramic material.

7. Sterile article with a joint prosthesis system part as claimed in at least one of the claims 1 to 6 which is received in a sterile pack.

## Revendications

1. Pièce de système de prothèse articulaire pour système de prothèse articulaire modulaire, comportant :
- un insert (5) associé à une cuvette articulaire bipolaire et composé d'une matière plastique et qui est constitué avec un support de sphère et configuré pour être reçu de manière mobile dans une coque extérieure (6) de la cuvette articulaire bipolaire et
- une tête de sphère articulaire (4) qui est réalisée avec un cône interne, la tête de sphère articulaire (4) étant prémontée de manière mobile sous forme de tête enfichée dans le support de sphère de l'insert (5),
**caractérisée par** un élément intermédiaire (3) dans lequel un cône extérieur est disposé de manière amovible dans le corps intérieur associé de la tête de sphère articulaire prémontée (4) et qui présente un cône intérieur destiné à recevoir un autre élément intermédiaire ou un col de prothèse (1).

2. Pièce de système de prothèse articulaire selon la revendication 1, **caractérisé en ce que** l'insert (5) est composé de polyéthylène.

3. Pièce de système de prothèse articulaire selon la revendication 1 ou 2, **caractérisé en ce que** le cône intérieur et le cône extérieur ne sont pas réalisés de manière coaxiale l'un par rapport à l'autre.

4. Pièce de système de prothèse articulaire selon au moins une des revendications précédentes, **caractérisé en ce que** l'élément intermédiaire (3) présente un élément de couplage qui est configuré pour se coupler à un outil d'échange.

5. Pièce de système de prothèse articulaire selon au moins une des revendications précédentes, **caractérisé en ce que** le cône intérieur est réalisé au niveau de l'élément intermédiaire (3) dans un trou borgne.

6. Pièce de système de prothèse articulaire selon au moins une des revendications précédentes, **caractérisé en ce que** la tête de sphère articulaire (4) est en matériau céramique.

7. Article stérile comportant une pièce de système de prothèse articulaire selon au moins une des revendications 1 à 6, qui est reçu dans un emballage stérile.
